# EUROPEAN PATENT SPECIFICATION

(11) **EP 3 359 233 B1**
(45) Date of publication and mention of the grant of the patent: **02.12.2020**
(21) Application number: 16775747.5
(22) Date of filing: 05.10.2016
(51) Int. Cl.: A61M 5/42, A61M 5/315, G16H 20/17

(54) **A POSITION DETERMINATION DEVICE FOR DETERMINING AND REGISTERING AN INJECTION LOCATION**
POSITIONSBESTIMMUNGSVORRICHTUNG ZUR BESTIMMUNG UND REGISTRIERUNG EINER INJEKTIONSSTELLE
DISPOSITIF DE DÉTERMINATION DE POSITION POUR DÉTERMINER ET ENREGISTRER UN EMPLACEMENT D'INJECTION

(30) Priority: 06.10.2015 DK 201570627
(43) Date of publication of application: 15.08.2018
(73) Proprietor: ROTO Health ApS, 3460 Birkerød (DK)
(72) Inventor: BØGGILD-DAMKVIST, David Tobias, 3650 Ølstykke (DK); TJALK-BØGGILD, Rasmus Thomas, 3100 Hornbæk (DK)
(74) Representative: Guardian IP Consulting I/S
(86) International application number: PCT/EP2016/073756
(87) International publication number: WO 2017/060281

(56) References cited:
- WO-A1-2014/023763
- WO-A1-2014/161952
- WO-A1-2015/085019
- WO-A1-2015/136564

## Description

### Field of the Invention

The invention relates generally to embodiments of a position determination device for and method of determining and registering an injection location of self-administered fluid drug administration on a user. Further the invention relates to calibration of such position determination devices.

### Background

A growing number of people have a condition involving fluid drug delivery administration where the user themselves deliver the relevant fluid drug (i.e. self-administration) by injection at an appropriate injection location or site using an injection device or the like.

Preferably, the fluid drug to be delivered is insulin but the invention is applicable to all fluid drug delivery devices where a user administers a drug on at least a somewhat regular basis, e.g. several times a day or less frequent like a number of times in a week, etc., by injection into an appropriate body part.

Other examples of user-administered fluid drugs includes MS therapies with interferons and immune suppressives (e.g. like Avonex, Rebif, Copaxone etc.), different hormone therapies (growth hormone, anti-/hormone followup treatments to breast or prostate cancer, etc.).

In such recurring self-injection regimens it is very important that the user does not inject the drug at the same location every time or too often as this may cause 'pockets' of the drug to gather under the skin, cause hard lumps and/or cause extra fat deposits to develop as well as the onset of lipohypertrophy and/or similar skin/tissue damages (that may change the way the liquid drug is absorbed by the body at that location), and so on. Therefore educating the user in injection techniques is often an important part of such treatment regimens and it is often recommended to change/rotate between injections locations. Furthermore, it may be recommended to inject not closer than 3 or even 1 cm (or some other distance(s)) of a previous injection location; at least within a given timeframe. To remember and keep track of the injections, users may use certain memory systems and/or simple schematics, but compliance has been shown to usually be lowered over time, in particular for user administered injection schemes.

A typical example of patient education material is found e.g. at: http://www.cc.nih.gov/ccc/patient_education/pepubs/subq.pdf However, a user may still forget where the last injection was made and/or even if rotating, perhaps some body locations are still used more frequently than others.

Some users may also be young children, which may have a harder time sticking rigorously to a recommended rotation scheme and/or remembering it fully all the time.

There is therefore a need for improved injection location handling and/or management.

It would also be a benefit to provide precise data about actual injection location(s).

Document WO2015085019 discloses a position determination device in conjunction with performing injections.

### Summary

It is an object to alleviate at least one or more of the above mentioned drawbacks at least to an extent.

Furthermore, it is an object to provide improved injection location handling and/or management.

Additionally, it is an object to enable precise data relating to actual injection location(s).

The invention is defined in claim 1 .

Accordingly, in one aspect of the present invention, a position determination device for determining and registering an injection location of administered fluid drug administration on a user, wherein the position determination device comprises
- a position determination element adapted to determine a current location of the position determination device, and
- one or more processing units adapted to derive data representing an actual injection location based on a current location determined by the position determination element.

In this way, specific data is obtained of where and when one or more drug injections actually have been made. This enables improved injection location handling and/or management of the drug administration.

In some embodiments, the one or more processing units is/are adapted to derive the data representing an actual injection location based on a current location determined by the position determination element in response to the user administering the fluid drug using a fluid drug administration device.

In some embodiments, the position determination device is further adapted to be secured to the fluid drug administration device.

In some embodiments, the position determination device has a generally hollow cylindrical shape with an opening or cut-out adapted to receive at least a part of the fluid drug administration device when being secured to this.

In some embodiments, the position determination device comprises a locking mechanism adapted to releasably engage with a mating or corresponding locking mechanism of the liquid drug administration device thereby attaching the position determination device at a predetermined location on and/or predetermined orientation in relation to the drug administration device.

In this way, it is ensured by such a locking mechanism that a distance from the position determination device to the needle (or another point of reference) of the drug administration device is known a priori. This avoids the need for re-calibration every time the position determination device is attached to the drug administration device.

In some further embodiments, the locking mechanism corresponds to a locking mechanism of a cap of the drug administration device.

In some further embodiments, the locking mechanism is further adapted to releasably engage with a mating or corresponding locking mechanism of a cap of the liquid drug administration device thereby enabling the cap to be attached to the position determination device (even when the position determination device is attached to the drug administration device).

In some embodiments, the position determination device further comprises a wireless communications unit adapted to automatically transmit the derived data representing an actual injection location to a separate electric device.

In some embodiments, an actual action of the user activating the fluid drug administration device to administer the fluid drug triggers deriving the data representing an actual injection location.

In some embodiments, a mechanical or remote sensor is attached directly to or attached in connection with an activation button of the fluid drug administration device where the mechanical or remote sensor is adapted to detect movement of the activation button of the fluid drug administration device.

A mechanical sensor may e.g. be physically attached or parallel to the activation button. A remote sensor would measure the distance to the activation button and determine any changes in the activation button position in relation to the position determination device.

In some embodiments, the position determination device further comprises a sound sensor adapted to register one or more distinct sounds caused by a user operating the drug administration device to increase, decrease, and/or release a drug dosage as an actual action of the user and wherein the position determination device is adapted to trigger deriving the data representing an actual injection location in response to registering the one or more distinct sounds.

Such distinct sounds are common among insulin administration devices and other types to allow users with impaired sight to hear when the dosage is respectively increased, decreased, or released.

In some embodiments, the position determination device further comprises
- a memory and/or storage comprising data representing at least one previous injection location,
- at least one indicator element, adapted to signal to the user
   ∘ that the current location of the position determination device, as determined by the position determination element, is at a location where it is acceptable or advisable to perform an injection taking into account at least the data representing at least one previous injection location, and/or
   ∘ that the current location of the position determination device, as determined by the position determination element, is at a location where it is not acceptable or advisable to perform an injection taking into account at least the data representing at least one previous injection location.

The position determination element comprises at least one gyroscope and/or at least one accelerometer.

In some embodiments, the position determination device is further adapted to register an actual amount of administered liquid drug and/or a time-stamp when deriving data representing an actual injection location.

In some embodiments, the position determination device further comprises an image sensor or other sensor element adapted to detect an amount of fluid drug being administered by the fluid drug administration device.

The position determination device is further adapted to
- register a starting position in response to the user moving the position determination device to a pre-determined starting position,
- register a location of an anchor position in response to the position determination element determining the movement from the starting position to the anchor position,
- register a location of the actual injection location in response to the position determination element determining the movement from the anchor position to the injection location.

In some embodiments, the position determination device is further adapted to
- recommend the anchor position and/or recommend the location of the actual injection location in response to a rotation schema taking into account one or more previously registered actual injection locations.

The position determination device is further adapted to
- register a starting position when the user has moved the position determination device to a starting position,
- if used, registering a region in connection with the starting position defining a region suitable for receiving injections,
- registering one or more anchor points, by determining their relative position in relation to the starting position, and for each anchor point registering a region defining a region associated with the given anchor point being suitable for receiving injections.

In some embodiments, the fluid drug is one selected from the group consisting of: insulin, drugs for multiple sclerosis therapies with interferons and immune suppressives (e.g. like Avonex, Rebif, Copaxone etc.), drugs for hormone therapies, drugs for growth hormone therapies, and drugs for anti-/hormone follow-up treatments to breast or prostate cancer.

In some embodiments, the position determination device is integrated with a fluid drug administration device and wherein the derived data representing an actual injection location is made available to at least another electric element of the fluid drug administration device.

In some embodiments, the position determination device is for determining and registering an injection location of self-administered fluid drug administration on a user.

In some embodiments, the position determination device is for determining and registering an injection location of administered fluid drug administration on a user administered by a medical professional.

### Brief description of the drawings

Figure 1 illustrates a schematic block diagram of embodiments of a position determination device;
Figures 2a - 2d schematically illustrate different views of one exemplary embodiment of a position determination device e.g. as illustrated in Figure 1;
Figure 3a schematically illustrates a perspective view of the position determination device of Figures 2a - 2d being secured to a fluid drug administration device;
Figure 3b schematically illustrates a perspective view of the position determination device and the fluid drug administration device of Figure 3a before the position determination device is secured to the fluid drug administration device;
Figure 4 schematically illustrates a flow chart of one embodiment of position determination by a position determination device as shown in Figures 1 to 3b and embodiments thereof;
Figure 5 schematically illustrates a flow chart of one embodiment of a method of suggesting an injection location;
Figure 6 schematically illustrates a flow chart of one embodiment of a calibration method of a position determination device, e.g. as shown in Figures 1 to 3b;
Figure 7 schematically illustrates a separate electric device displaying a 'heatmap' view or similar of earlier injections;
Figure 8 schematically illustrates an embodiment of a locking mechanism for attaching an embodiment of a position determination device to a drug administration device; and
Figures 9a and 9b schematically illustrate an embodiment of the position determination device attached to a drug administration device and by itself.

### Detailed description

Various aspects and embodiments of a position determination device for and methods of determining and registering an injection location of administered fluid drug administration on a user and calibration of such position determination devices as disclosed herein will now be described with reference to the figures.

When relative expressions such as "upper" and "lower", "right" and "left", "horizontal" and "vertical", "clockwise" and "counter clockwise" or similar are used in the following terms, these only refer to the appended figures and not to an actual situation of use. The shown figures are schematic representations for which reason the configuration of the different structures as well as their relative dimensions are intended to serve illustrative purposes only.

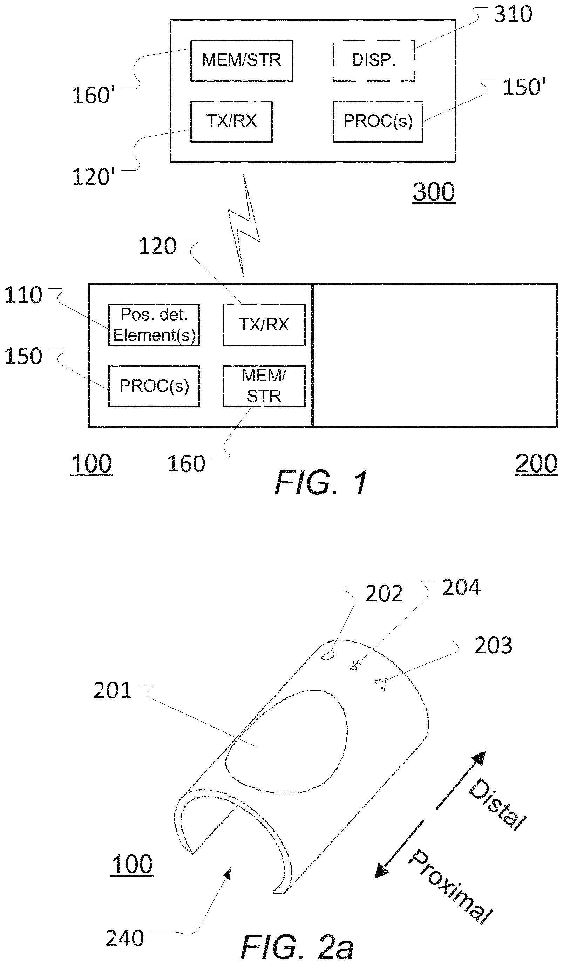

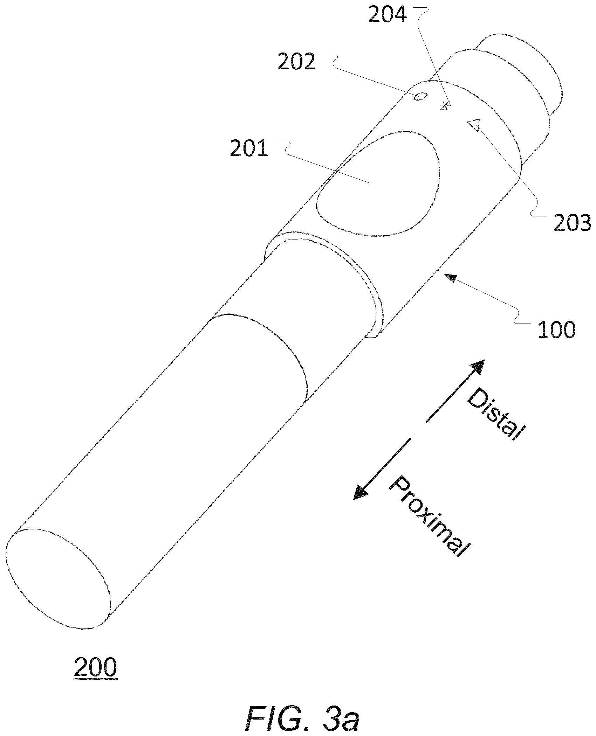

In that context it may be convenient to define that the term "proximal direction" in the appended figures is meant to refer to the direction of the position determination device (and its associated or integrated fluid drug administration device) that normally, during use, would point towards an injection element of the fluid drug administration device , as depicted e.g. in Figures 2a and 3a - 3b, whereas the term "distal direction" is meant to refer to a direction parallel and opposite to the proximal direction.

Figure 1 illustrates a schematic block diagram of embodiments of a position determination device.

Shown is one embodiment of a position determination device 100 and a fluid drug administration device 200. The position determination device 100 may e.g. be integrated with the fluid drug administration device 200 or alternatively be attached or secured to the fluid drug administration device 200, e.g. as shown and explained further in connection with Figures 3a - 3b and 8 - 9.

The fluid drug administration device 200 may be of any suitable type that is used by a user or a medical professional to administer a fluid drug at an appropriate injection location on a given user. The fluid drug administration device 200 may e.g. be an insulin administration device such as an insulin pen, insulin doser, insulin pump, etc. Alternatively, the fluid drug administration device may e.g. be a syringe for delivery of interferons, immune suppressives, or hormone therapies.

The position determination device 100 comprises one or more position determination elements 110 and in this particular and other embodiments, one or more processing units 150, at least one digital memory and/or storage 160, and at least one communications unit 120, preferably one or more standardised wireless communications units e.g. like Wi-Fi, Bluetooth, NFC, etc. communications units.

If the position determination device 100 is integrated with the fluid drug administration device 200 they may share one or more elements, such as the processing unit(s), the digital memory/storage, and/or the communication unit(s).

Further shown is a separate electric device 300 comprising its own one or more processing units 150', at least one digital memory and/or storage 160', at least one communications unit 120', and optionally a display 310.

Preferably, the position determination device 100 (and/or the fluid drug administration device 200) may wirelessly communicate or exchange data and information with the separate electric device 300.

The separate electric device 300 may e.g. be a mobile communications and/or computation device such as a smartphone, a tablet, a laptop or PC, etc. or a separate dedicated special purpose device, such as a data-logger or the like.

The position determination device 100 is for determining and registering an injection location of (e.g. self-administered) fluid drug administration on a user as will be explained in the following.

According to an aspect of the present invention, the position determination element 110 is adapted to determine, during use, a current location of the position determination device 100 and the one or more processing units 150 is/are adapted to derive, during use, data representing an actual injection location based on a current location determined by the position determination element 110.

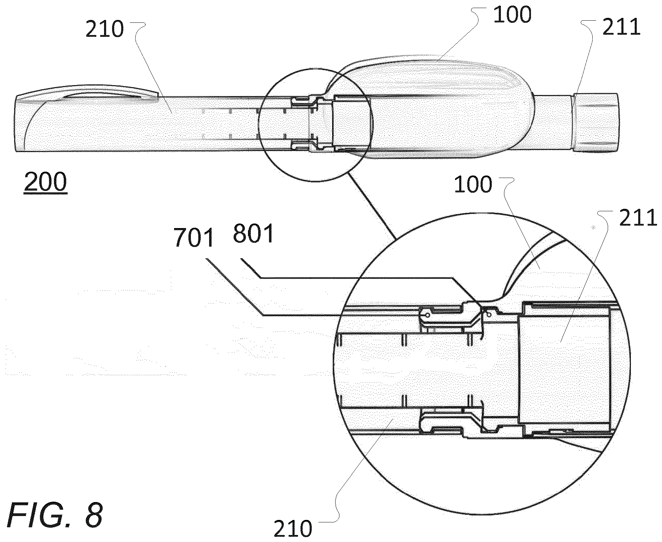

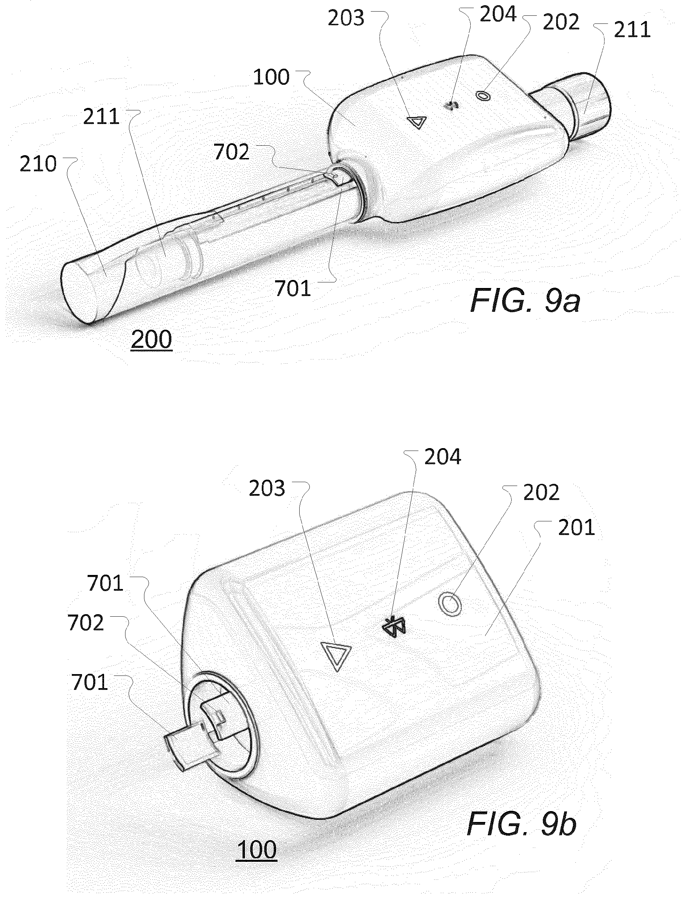

According to a further aspect of the present invention, the position determination device 100 may be attached or secured and (releasably) locked in relation to the drug administration device 200 with the use of a locking mechanism according to various embodiments (see e.g. 701 in Figures 8 and 9 for one embodiment) at a predetermined location on and/or predetermined orientation in relation to the drug administration device 200. In this way, it is ensured by such a locking mechanism that a distance from the position determination device 100 to the needle of the drug administration device 200 is always known a priori. This avoids the need for re-calibration every time the position determination device 100 is attached to the drug administration device 200.

In some further embodiments, the position determination device 100 attaches to the drug administration device 200 like a cap of the drug administration device 200 normally would when the position determination device 100 is not present, i.e. the locking mechanism of the position determination device 100 corresponds to the locking mechanism of the cap in that they will be the same or at least similar or compatible. This ensures that the position determination device 100 can be attached or secured to the drug administration device 100 in a reliable way, since the locking mechanism position determination device 100 is a 'copy' of the locking mechanism of the cap.

In some further embodiments, the position determination device 100 is further adapted to allow the cap of the drug administration device 200 to be attached to the position determination device 100 rather than directly to the drug administration device 200 itself when the position determination device 100 is attached or secured to the drug administration device 200. This ensures that the cap may still be attached thereby protecting the relevant parts of the drug administration device 200 in a usual way.

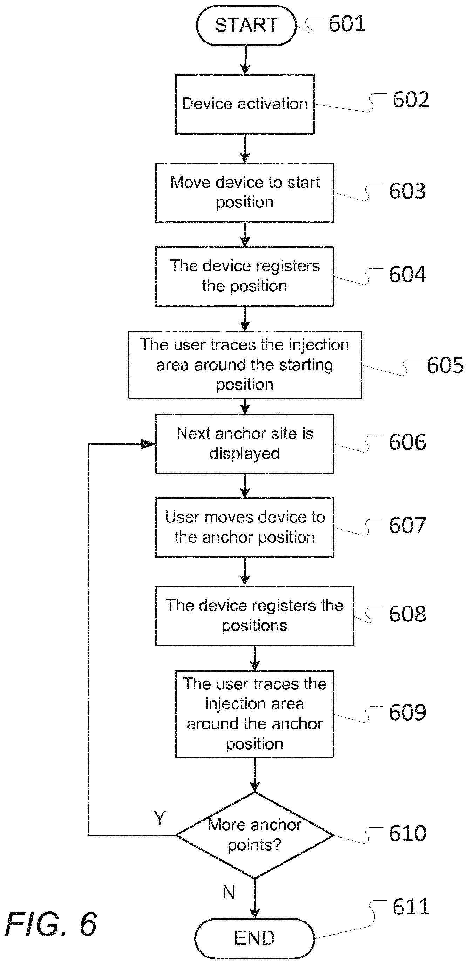

The actual injection location may e.g. be calculated in the following manner: From a known starting position the position determination element 110 is reset to represent a zero-length movement vector. As the position determination device 100 is moved, the position determination element 110 update its internal representation of the distance moved to produce a 3D movement vector. When the position determination device 100 is moved to an anchor point, this movement vector is compared to the calibrated vectors to determine which anchor point the position determination device 100 is at. In addition, data from the position determination element 110 may e.g. be used to determine the relative position in relation to the torso, or the limb where the anchor point is located. The border of the injection area around the anchor points is calibrated e.g. as described in connection with Figure 6. Using this calibrated area, then the anchor position, the starting position, the exact position of the injection site may be determined when the position determination element 100 (and e.g. the fluid drug administration device 200 as attached or integrated) is moved by the user to a final injection site.

In this way, specific data is obtained of where (and when) one or more drug injections actually have been made. This enables improved injection location handling and/or self-management of the drug administration including e.g. using knowledge of previous injections locations to recommend the next appropriate injection location taking this knowledge into account - e.g. to adhere to and comply with a rotation scheme, a regimen, schedules for administering the drug, etc. and to improve drug absorption and avoid the drawbacks mentioned earlier, to actively warn a user if the user is about to use an injection location that is too close to an earlier injection location (e.g. within a given time-frame), and further on, as will be explained further in the following.

In some embodiments, the position determination device 100 may automatically transmit, using the wireless communications unit 120, the derived data representing an actual injection location to the separate electric device 300 and/or the fluid drug administration device 200. In this way, the data relating to actual injection is readily made available and may be used for various purposes e.g. as disclosed herein.

In some embodiments, the data representing an actual injection location is derived in response to the user administering the fluid drug using the fluid drug administration device 200. This may in some embodiments be an actual action of the user activating the fluid drug administration device 200 to administer the fluid drug that triggers deriving the data representing an actual injection location.

In some embodiments, the position determination device 100 may also comprise at least one indicator element (see e.g. 202, 203, and 204 in Figures 2a - 3b and 8 - 9).

The at least one indicator element (e.g. light, LED, sound emitter, etc.) may be adapted to signal to the user, during use, that a current location of the position determination device 100, as determined by the position determination element 110, is at a location where it is acceptable or advisable to perform an injection taking into account at least the data representing at least one previous injection location.

The at least one indicator element may alternatively or in addition be adapted to signal to the user, during use, that the current location of the position determination device 100, as determined by the position determination element 110, is at a location where it is not acceptable or advisable to perform an injection taking into account at least the data representing at least one previous injection location.

In addition or as an alternative, the separate electric device 300 and/or the fluid drug administration device 200 may comprise at least indicator element performing the same function(s).

One or more of the at least one indicator elements may also be graphical elements as part of a graphical user interface.

The position determination element 110 may e.g. comprise at least one gyroscope and/or at least one accelerometer to determine a location, e.g. in a relative way using fixed known/calibrated positions, as will be explained further in the following. Alternatively, other types of position determination elements may be e.g. including absolute measurement elements.

Figures 2a - 2d schematically illustrate different views of one exemplary embodiment of a position determination device e.g. as illustrated in Figure 1.

Shown in Figure 2a is one exemplary embodiment of a position determination device 100 corresponding, in function at least, to embodiments already described in connection with Figure 1. This particular embodiment is of a position determination device 100 that is adapted to be attached or secured, during use, to a fluid drug administration device, e.g. like 200 described in connection with Figure 1 and elsewhere.

The position determination device 100 may e.g. be adapted to be secured to the fluid drug administration device using one or more attachment elements or similar (not shown; see e.g. 701 in Figures 8 and 9).

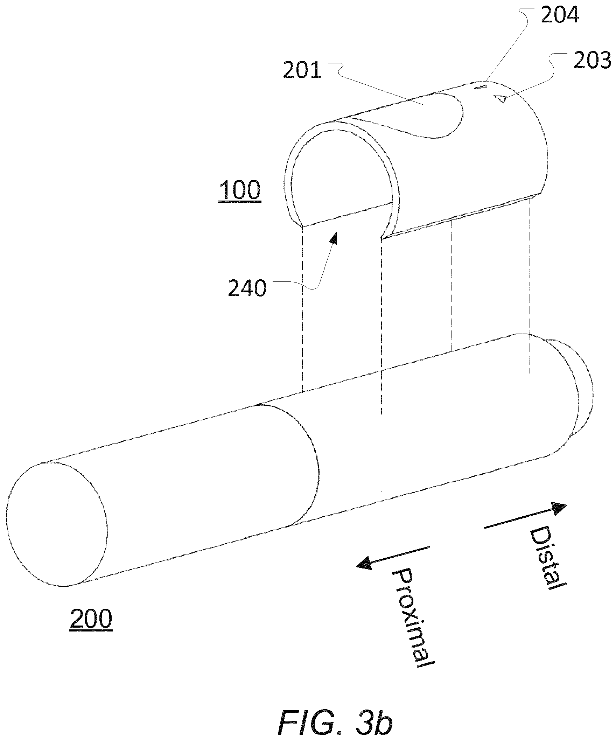

Alternatively, the position determination device 100 may be adapted to be secured to the fluid drug administration device due to its particular shape, e.g. such as shown in the figure and explained further in connection with Figures 3a and 3b.

In this and corresponding embodiments, the position determination device 100 comprises one activation or operation button or similar 201 and at least one indicator element 202, 203, 204 such as one or more lights, LEDs, etc. and/or one or more sound emitters used to signal the user of various indications, information, status, etc.

In the shown embodiment, the position determination device 100 comprises a confirmation light or LED 202, a warning light or LED 203, and a wireless communications connection light or LED 204.

The confirmation light or LED 202 may indicate when a given position is ok for drug injection, while the warning light or LED 203 may indicate when a given position is not ok. The connection light or LED 204 may indicate when a wireless connection is established to a separate electric device (e.g. like 300 in Figure 1).

It should be noted, that the functions of two or more lights or LEDs could be done by fewer lights or LEDs, e.g. only a single one. As an example, one multi-coloured light or LED could be e.g. green for confirmation and red for warning. In addition or as an alternative, the indications could also be audible e.g. using one or more sound emitters.

The indications could also be given on a separate electric device (e.g. like 300 Figure 1) e.g. in an app or program running on a smartphone, tablet, laptop, PC, etc. instead or in duplication.

Also indicated in Figure 2a is a proximal and a distal direction. The proximal direction is a direction generally towards the end of the position determination device 100 that normally would point to the injection element of a fluid drug administration device (see e.g. 200 in Figures 1 and 3a - 3b) that the position determination device 100 is secured to while the distal direction is parallel and opposite to the proximal direction.

It should be noted, that the orientation of the position determination device 100 when secured to the fluid drug administration device in certain embodiments may not be significant. The shown embodiment of the position determination device 100 could e.g. be rotated about its central axis on the fluid drug administration device and it could e.g. also be turned 180° (so the distal end of the position determination device 100 would be in the proximal direction of the fluid drug administration device) while still working.

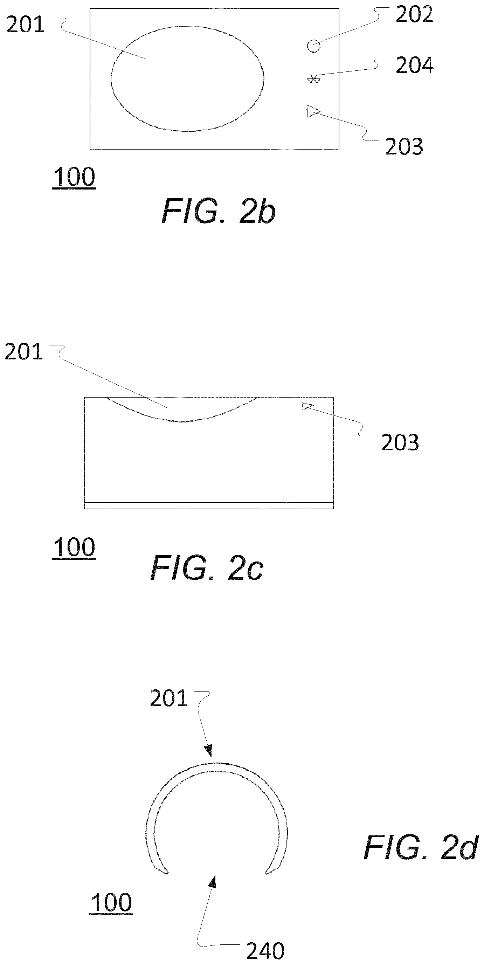

Shown in Figure 2b is the position determination device 100 of Figure 2a shown from 'above', i.e. shown from a direction pointing towards the activation or operation button 201 and the indicators 202, 203, and 204.

Shown in Figure 2c is the position determination device 100 of Figure 2a shown from one side.

Shown in Figure 2d is the position determination device 100 of Figure 2a shown from one end. As can be seen, the position determination device 100 in this view has a more or less circular ring shape with an opening or cut-out 240 for receiving at least a part of the fluid drug administration device when being secured to this.

The position determination device 100 may e.g. have a suitable 'soft' or flexible material on its inside on parts that are to be in contact with the fluid drug administration device when attached thereto.

Figure 3a schematically illustrates a perspective view of the position determination device of Figures 2a - 2d being secured to a fluid drug administration device.

Shown is the position determination device 100 of Figure 2a in a state where it is attached or secured to a fluid drug administration device 200 that e.g. may be of the type mentioned in connection with Figure 1, and in particular a liquid insulin administration device such as an insulin pen or similar.

The fluid drug administration device 200 may be of any suitable type and in this and corresponding embodiments of the position determination device 100 it does not require any modification of the fluid drug administration device to take advantage of the present invention. Moving and using the fluid drug administration device 200 will move the position determination device 100 with a fixed positional relationship between them. If the position determination device 100 is removed and attached back onto the fluid drug administration device 200 at another position there may in certain cases be a need for re-calibration, unless the position and/or orientation of the position determination device 100 on the fluid drug administration device 200 is known apriori as disclosed herein.

Figure 3b schematically illustrates a perspective view of the position determination device 100 and the fluid drug administration device 200 of Figure 3a before the position determination device 100 is secured to the fluid drug administration device 200.

In this and corresponding embodiments, the position determination device 100 is simply snapped or clicked onto the fluid drug administration device 200 as indicated by the four broken lines. The shape of the position determination device 100 being generally of a hollow cylindrical shape with its opening or cut-out - as also shown in Figures 2a - 2d - enables the position determination device 100 to easily but securely be temporarily attached or fixed to the fluid drug administration device 200.

The specific shape of the position determination device 100 of Figure 3b and corresponding devices are particularly well suited to be secured to pen-type fluid drug administration devices. But the shape may of course vary according to the fluid drug administration device that it is to be used together with.

Alternatively, the position determination device 100 may be 'slided' onto the fluid drug administration device 200 from one of the ends. As other alternatives, the position determination device 100 may comprise one or more attachment or securing elements enabling it to be secured to the fluid drug administration device 200 in other ways (whereby the position determination device 100 may have a different shape) e.g. as described in connection with Figures 8 and 9.

The fluid drug administration device 200 will typically comprise at least one activation button or other activation elements to initiate the actual administration of the fluid drug to the user.

In some embodiments, the position determination device 100 will derive data representing an actual injection location based on the user specifically activating the fluid drug administration device 200 to administer the fluid drug. In this way, only one activation action from the user is needed even if the position determination device 100 and the fluid drug administration device 200 are separate devices.

If the position determination device 100 is integrated with the fluid drug administration device 200 this is of course relatively easily enabled.

Even if the position determination device 100 is not integrated with the fluid drug administration device 200 an alternative measure may e.g. be that pressing the activation button or the like on the fluid drug administration device 200 physically will activate the activation button of the position determination device at the same time. It may also be the other way around where activation of the activation button of the position determination device physically will activate an activation button or the like on the fluid drug administration device 200.

This could e.g. be achieved if the position determination device 100 is located on the fluid drug administration device 200 so that the position determination device's activation button is on top of (or in any other engaging relationship) the activation button of the drug administration device 200. It is to be understood that the specific location of the various activation buttons may be different in specific implementations.

Alternatively, a pressure sensor may e.g. be connected to the activation button of the fluid drug administration device 200 to allow the position determination device 100 to register the position at the exact moment of the injection. This pressure sensor may be manual, mechanical (attached to or parallel to the 200 activation mechanism) or may be implemented using a remote distance measurement (like an IR light source and a camera or otherwise). This distance measurement would be used to detect movement of the activation button of the fluid drug administration device 200.

A microphone or other sound sensor may also be used as another alternative for those drug administration devices 200 that incorporates distinct sounds for increase, decrease, and release of the drug dosage. This is common among insulin administration devices to allow users with impaired sight to hear when the dosage is respectively increased, decreased, or released.

In some embodiments (including the ones explained in connection with Figures 1 to 2d), the position determination device 100 may further comprises an image sensor or other sensor element adapted to detect an amount of fluid drug being administered by the fluid drug administration device 200. In this way, dosage data may be collected (and communicated further on) by the position determination device 100 in situations where a fluid drug administration device 200 otherwise does not support this.

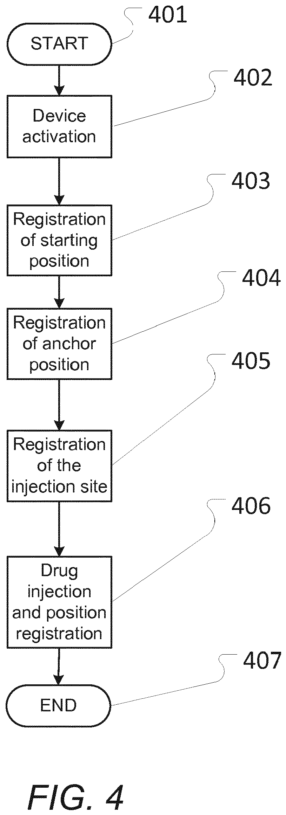

Figure 4 schematically illustrates a flow chart of one embodiment of position determination by a position determination device as shown in Figures 1 to 3b and embodiments thereof.

The method starts or initiates at step 401 and at step 402 the user activates the position determination device (e.g. the position determination device 100 shown and explained in connection with Figures 1 - 3b and variations thereof) by pressing an appropriate activation element (e.g. like 201 shown in Figures 2a - 3b).

At step 403, the user moves the position determination device, or rather moves the fluid drug administration device (e.g. like 200 shown and explained in connection with Figures 1 and 3a - 3b) with the attached or integrated position determination device to a given pre-determined or pre-selected starting position. The starting position may e.g. be the navel, a knee, an elbow or any other appropriate starting position as calibrated and may be used as the starting point of any location determination for the associated drug injection. The starting position may e.g. be selected by the user.

After a few seconds of non-movement (or by the user activating an appropriate activation element or some other action or means), the current position (being the starting position) is automatically registered and stored as data in the memory and/or storage of the position determination device.

For relevant embodiments, at least one indicator element, e.g. a confirmation light or LED like 202 in Figures 2a - 3b, is activated until the position determination device 100 (and the fluid drug administration device) is moved again.

At step 404, the position determination device (and the fluid drug administration device) is moved to another location referred to as an anchor point or the like related to a desired injection location. An anchor point may e.g. be the left knee of the user if the desired injection location is at the left thigh, etc.

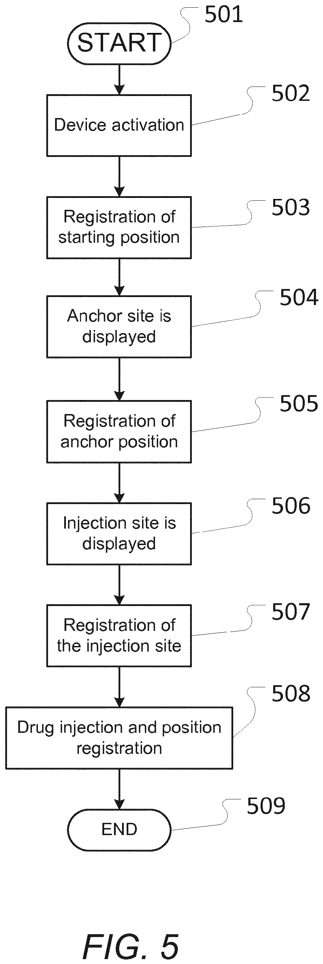

The anchor point location may e.g. be chosen by the user or suggested or recommended e.g. as described in connection with step 504 of Figure 5.

After a few seconds of non-movement (or by the user activating an appropriate activation element), the current position (being the anchor point) is automatically registered and stored as data in the memory and/or storage of the position determination device.

For relevant embodiments, the least one indicator element, e.g. the confirmation light or LED, is activated until the position determination device (and the fluid drug administration device) is moved again.

In some embodiments, the anchor point is not necessarily needed and step 403 proceeds directly to step 405. This depends on the used position determination scheme.

At step 405, the position determination device (and the fluid drug administration device) is moved to a desired injection location. The desired injection location may e.g. be chosen by the user or suggested or recommended e.g. as described in connection with step 506 of Figure 5.

If the position of the desired injection location is determined to be too close to previous injection locations (e.g. within about 1 or about 3 cm as advised in the current state of the diabetes treatment art within a given time-frame) by the one or more processing units of the position determination device according to overall general or the user's specifically entered guidelines, then at least one indicator element, e.g. a warning light or LED like 203 in Figures 2a - 3b, will activate prompting the user that current position is not suitable or recommended for injection and that another injection location should be chosen.

The position determination device (and the fluid drug administration device) should then be moved to a new injection location and when that is done and the new injection location is determined - by the one or more processing units like described above - to be suitable then least one indicator element, e.g. a confirmation light or LED like 202 in Figures 2a - 3b, is activated to signal that it is ok to perform an injection at the current injection location.

At step 406, the user activates the fluid drug administration device to administer the fluid drug at the injection location and also presses an appropriate activation element (e.g. 201 shown in Figures 2a - 3b) to register and store the position of the actual injection location as data in the memory and/or storage of the position determination device. As noted previously, it may alternatively be the activation of the fluid drug administration device that triggers the registration and storage of the actual injection location.

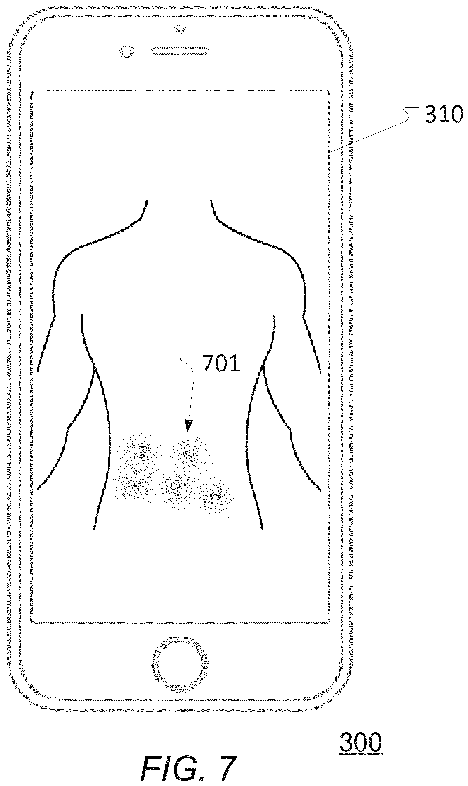

Data representing the position of the actual injection location is then transmitted to a separate electric device (e.g. 300 in Figures 1 and 7) and stored there for later use and/or retrieval. After the transmission is completed the position determination device deactivates and the method ends at step 407.

Data representing a number of actual positions may in this way be collected, e.g. together with additional information, time-stamp, amount of administered liquid drug (for embodiments supporting that), etc. This provides a very comprehensive 'diary' for the user, which may be useful for a user in improving self-administration routines as well as for medical professionals e.g. to check for regimen compliance, gather data for research, improve patient education, etc.

Using both a start point and an anchor point increases the precision of the registration of the injection location. However, in in some alternative embodiments, an anchor point may be selected in some way (e.g. by the user choosing one or by recommending one) and the injection location is determined relative only to the selected anchor point, i.e. a starting point is not used.

Figure 5 schematically illustrates a flow chart of one embodiment of a method of suggesting an injection location.

The method starts or initiates at step 501 and at step 502 (which may correspond to step 402 in Figure 4) the user activates the position determination device (e.g. the position determination device 100 shown and explained in connection with Figures 1 - 3b and variations thereof) by pressing an appropriate activation element (e.g. 201 shown in Figures 2a - 3b).

At step 503 (which may correspond to step 403 in Figure 4), the user moves the position determination device and the fluid drug administration device (e.g. 200 shown and explained in connection with Figures 1 and 3a - 3b) to a given pre-determined or pre-selected starting position, e.g. like the navel, a knee, an elbow, or any other appropriate starting position as calibrated.

After a few seconds of non-movement (or by some other action or means), the current position (being the starting position) is automatically registered and stored as data in the memory and/or storage of the position determination device. For relevant embodiments, at least one indicator element, e.g. a confirmation light or LED like 202 in Figures 2a - 3b, is activated until the position determination device 100 (and the fluid drug administration device) is moved again.

At step 504, an appropriate injection location to use is derived.

The injection location to use this time may be derived by a separate electric device (e.g. 300 in Figures 1 and 7). Alternatively, it may be derived by the one or more processing units of the position determination device or a fluid drug administration unit.

The inject location to use next is derived according to general or the user's specifically entered guidelines. This may for example be by a scheme where the determination of injection sites follow a circular or spiral pattern across the belly to ensure an even spread of sites over a given period like a week, for example a linear upward moving of sites on the thighs alternating between the right and the left, or a time-based guideline always advising about the injection site unused for the longest amount of time to ensure the largest spread. The guidelines used should be chosen based on dialogue with medical professionals and individual preferences.

After the next injection location to use has been derived, an anchor point associated with the given next injection location is then presented to the user, e.g. on a display of the separate electric device or in another suitable way.

At step 505, the user moves the fluid drug administration device (and thereby the position determination device) to the presented anchor point or the like of the suggest injection location. If the user moves the fluid administration device and the position determination device to a different location than the suggested position, a warning may e.g. displayed to the user (e.g. using at least one indicator element such as a warning light or LED like 203 in Figures 2a - 3b) and/or on a separate electric device (e.g. 300 in Figures 1 and 7).

After a few seconds of non-movement (or by some other action or means), the current position (being the anchor point) is automatically registered and stored as data in the memory and/or storage of the position determination device. For relevant embodiments, the least one indicator element, e.g. the confirmation light or LED, is activated until the position determination device (and the fluid drug administration device) is moved again.

In some embodiments, the anchor point is not necessarily needed and step 503 proceeds directly to step 506. This depends on the used position determination scheme

At step 506, the injection location (as being associated with the registered anchor point) to use next as derived at step 504 is displayed e.g. on the display of the separate electric device or in another suitable way.

At step 507, the user moves the fluid drug administration device (and thereby the position determination device) to the presented suggested injection location.

For relevant embodiments, at least one indicator element, e.g. a warning light or LED like 203 in Figures 2a - 3b, will activate and stay activated until the position determination device is moved within a predetermined threshold distance of the injection location that was displayed at step 506.

The predetermined threshold may be defined in the user's entered guidelines or be an overall general threshold.

For relevant embodiments, when the position determination device comes within the predetermined threshold distance of the injection location then least one indicator element, e.g. a confirmation light or LED like 202 in Figures 2a - 3b, is activated to signal that it is ok to perform an injection at the current injection location.

At step 508, the user activates the fluid drug administration device to administer the fluid drug at the injection location and also presses an appropriate activation element (e.g. 201 shown in Figures 2a - 3b) to register and store the position of the actual injection location as data in the memory and/or storage of the position determination device. As noted previously, it may alternatively be the activation of the fluid drug administration device that triggers the registration and storage of the actual injection location.

Data representing the position of the actual injection location is then transmitted to the separate electric device (e.g. 300 in Figures 1 and 7) and stored there for later use and/or retrieval. After the transmission is completed the position determination device deactivates and the method ends at step 509.

Figure 6 schematically illustrates a flow chart of one embodiment of a calibration method of a position determination device.

The method starts or initiates at step 601 and at step 602 the user activates the position determination device (e.g. the position determination device 100 shown and explained in connection with Figures 1 - 3b and variations thereof) by pressing an appropriate activation element (e.g. 201 shown in Figures 2a - 3b).

At step 603, the user moves the position determination device, or rather moves the fluid drug administration device (e.g. 200 shown and explained in connection with Figures 1 and 3a - 3b) with the attached or integrated position determination device to a given pre-determined or pre-selected starting position and keeps the position determination device still. It may be that the user chooses the starting position him- or herself or that it is predefined and presented in some way, e.g. using a separate electric device (e.g. 300 in Figures 1 and 7).

This starting position is intended to be used for the following position determinations (e.g. at least until a next (re-)calibration.

At step 604, the user activates an appropriate activation element and the current position (being the starting position) is then registered and stored as data in the memory and/or storage of the position determination device.

At step, 605 the user indicates the boundary of the injection area that is to be associated with the starting position (if there is one to be associated). In some embodiments, this may be done by the user moving the position determination device from the starting position to an edge of the injection area and then while keeping the activation element active or pressed, the user traces the edge of the injection area with the position determination device (being attached or integrated with the fluid drug administration device). Alternatively, other ways to indicate the boundary and/or the injection area may be used.

The positon determination device then registers and stores the injection area and/or boundary as data in its memory and/or storage and transmits the data to the separate electric device (e.g. 300 in Figure 1) for storage and/or visual feedback to the user.

At step 606, the separate electric device displays information to the user about which anchor position to register.

At step 607, the user moves the position determination device and the fluid drug administration device to the shown anchor position.

At step 608, the current position (being the anchor point) is automatically registered and stored as data in the memory and/or storage of the position determination device e.g. by keeping the position determination device still for a predetermined amount or time or by activating an appropriate activation element (e.g. 201 shown in Figures 2a - 3b). For relevant embodiments, the least one indicator element, e.g. the confirmation light or LED, is activated until the position determination device (and the fluid drug administration device) is moved again.

At step 609, the user indicates the boundary of the injection area that is to be associated with the current anchor position and it is registered and stored in the same manner as at step 605 for the starting position.

At step 610 it is checked whether this procedure is to be done for any further anchor points. If yes, the method loops back to step 606 whereby steps 606 - 610 is repeated in connection with the next anchor point. If no (signifying no remaining anchor points), the calibration process terminates at step 611.

In this way, simple and reliable calibration is provided.

Figure 7 schematically illustrates a separate electric device displaying a 'heatmap' view or similar of earlier injections.

Shown is a separate electric device 300, e.g. corresponding to the one shown in Figure 1, having a display 310 to be used according to some aspects in connection with some embodiments of a position determination device (such as 100 shown in Figures 1 - 3b, 8 - 9, and variations thereof) and a fluid drug administration device (such as 200 shown in Figures 1 and 3a - 3b, 8 - 9, and variations thereof).

As already mentioned, actual injections locations may preferably be registered on an ongoing basis using the position determination device and the fluid drug administration device.

According to these aspects, the actual injection locations may be shown as a graphical representation of data generally known as a 'heat map' or similar where the individual values (location e.g. together with further relevant information like time of injection, actual amount of injected fluid drug, etc.) are represented as colors in a color scheme superimposed on a picture or a graphic representation of the overall injection area(s), e.g. like the torso, the thighs, upper arms, or what other injection area the user has defined, etc. The values may e.g. be stored in a matrix data structure and/or any other suitable data structure.

The color scheme may e.g. be one from green to red where a given injection location is marked yellow if it was taken in (too) close proximity (e.g. within 1 or 3 cm, or as chosen by the user) to one another injection location made within a predetermined time (e.g. a week or other timeframe as e.g. determined by the user), and red if it is in (too) close proximity to 2 or more injection locations, and green in all other cases.

The heatmap may be updated from the device when activated thus enabling the user to check an intended injection location in relation to previous injection locations.

In a recommendation mode (e.g. as indicated in Figure 5), the device itself may show status of injection locations based om the same color scheme or recommend locations based on the chosen rotation principles.

The aspect(s) of showing previous injection locations like this may be used independently of the described aspects and embodiments of the position determination device although they together work especially well.

Figure 8 schematically illustrates an embodiment of a locking mechanism for attaching an embodiment of a position determination device to a drug administration device.

Shown are an embodiment of a position determination device 100 as disclosed herein being attached to a drug administration device, here in the form of a insulin pen or other pen type fluid drug administration device, as well as an enlargement illustrating further details.

In this and corresponding embodiments, the position determination device 100 as disclosed herein comprises one exemplary embodiment of an attachment element in the form of a locking mechanism 701 adapted to releasably engage with a mating or corresponding locking mechanism 801 of the liquid drug administration device 200. The shown drug administration device 200 comprises a cap 210 and a main body 211. More specifically, the locking mechanism 701 is adapted to engage with a locking mechanism 801 of the main body 211 of the drug administration device 200 that normally is used to engage a locking mechanism of the cap 210, i.e. the locking mechanism 701 of the position determination device 100 corresponds to the locking mechanism of the cap in that they will be the same or at least similar or compatible.

In some further embodiments, and as shown, the locking mechanism 701 is further adapted to engage with the locking mechanism of the cap 210 (see e.g. 702 in Figure 9) so that the cap 210 still can be attached to the drug administration device 200 even with the position determination device 100 in place, now just connecting with the position determination device 100 instead of directly to the drug administration device 200.

The locking mechanism 701 allows the user an easy way to attach / detach the position determination device 100 to / from the drug administration device 200 in a reliable way. The specific embodiment of such a locking mechanism 701 will typically depend on the specific shape, size, and form of the cap 210 of the drug administration device 200 and how this attaches to the main body of the drug administration device 200.

Figures 9a and 9b schematically illustrate an embodiment of the position determination device attached to a drug administration device and by itself.

Shown in Figure 9a is illustrated an embodiment of the position determination device 100 attached to a drug administration device 200 as disclosed herein where the cap 210 of the drug administration device 200 is also attached to the main body 211 of the drug administration device 200. The cap 210 is shown transparently or with a cut-out to illustrate parts of the main body 211 of the drug administration device 200. The attachment is done using a locking mechanism 701 as disclosed herein. In some embodiments of the locking mechanism 701 and as shown it comprises an engaging element 702 for allowing a locking mechanism of the cap 210 to engage so that the cap 210 may be attached to the position determination device 100 instead of to the main body 211 of the drug administration device 200.

The shown drug administration device 200 also comprises an activation button 201, as well as a number of indicator elements here in the form of a confirmation light or LED 202, a warning light or LED 203, and a wireless communications connection light or LED 204 for user feedback as disclosed herein.

In some embodiments as disclosed herein, the position determination device further comprises a temperature sensor adapted to continuously monitor whether the liquid drug is kept at safe temperature. A warning may be given before the temperature reaches levels that will make the liquid drug unfit for use. This warning may be given using audiovisual cues and/or by the separate electric device.

According to an alternate aspect of the invention, image recognition may be used as a method of locating injection sites.

Even though, the description mainly mention user-administrated injection where a person to receive the liquid drug is also the user to administer the drug it is to be understood that at least for certain aspects, the user may e.g. be a medical professional or the like.

## Claims

1. A position determination device (100) for determining and registering an injection location of administered fluid drug administration on a user, wherein the position determination device (100) comprises
- a position determination element (110) adapted to determine a current location of the position determination device (100), the position determination element (110) comprising at least one gyroscope and at least one accelerometer, and
- one or more processing units (150) adapted to derive data representing an actual injection location based on a current location determined by the position determination element (110),
wherein the position determination device (100) is adapted to
- register a starting position in response to the user moving the position determination device (100) to a pre-determined starting position,
**characterised in that** the position determination device (100) is further adapted to
- register a location of an anchor position in response to the position determination element (110) determining the movement from the starting position to the anchor position,
- register a location of the actual injection location in response to the position determination element (110) determining the movement from the anchor position to the injection location,
- reset the position determination element (110) to represent a starting movement vector at the starting position,
- updating an internal representation of the position determination element (110) to produce a three-dimensional movement vector in response to the distance moved from the starting position,
- when the position determination device (100) is moved to the anchor position then comparing the three-dimensional movement vector to calibrated vectors to determine which anchor position the position determination device (100) is located at, and
- determine an exact position of an injection site in response to the user moving the position determination element (110) from the anchor position to a final injection site.

2. The position determination device (100) according to claim 1, wherein the one or more processing units (150) are adapted to derive the data representing an actual injection location based on a current location determined by the position determination element (110) in response to the user administering the fluid drug using a fluid drug administration device (200).

3. The position determination device (100) according to any one of claims 1 - 2, wherein
- the position determination device (100) is further adapted to be secured to the fluid drug administration device (200).

4. The position determination device (100) according to claim 3, wherein the position determination device (100) has a generally hollow cylindrical shape with an opening or cut-out (240) adapted to receive at least a part of the fluid drug administration device (200) when being secured to this.

5. The position determination device (100) according to any one of claims 3 - 4, wherein the position determination device (100) comprises a locking mechanism (701) adapted to releasably engage with a mating or corresponding locking mechanism (801) of the liquid drug administration device (200) thereby attaching the position determination device (100) at a predetermined location on and/or predetermined orientation in relation to the drug administration device.

6. The position determination device (100) according to claim 5, wherein the locking mechanism (701) corresponds to a locking mechanism of a cap (210) of the drug administration device (200).

7. The position determination device (100) according to claim 5 or 6, wherein the locking mechanism (701) is further adapted to releasably engage with a mating or corresponding locking mechanism (801) of a cap (210) of the liquid drug administration device (200) thereby enabling the cap (210) to be attached to the position determination device (100).

8. The position determination device (100) according to any one of claims 1 - 7, wherein
- the position determination device (100) further comprises a wireless communications unit (120) adapted to automatically transmit the derived data representing an actual injection location to a separate electric device (200; 300).

9. The position determination device (100) according to any one of claims 1 - 8, wherein
- an actual action of the user activating the fluid drug administration device (200) to administer the fluid drug triggers deriving the data representing an actual injection location.

10. The position determination device (100) according to claim 9, wherein a mechanical or remote sensor is attached directly to or attached in connection with an activation button of the fluid drug administration device (200) where the mechanical or remote sensor is adapted to detect movement of the activation button of the fluid drug administration device (200).

11. The position determination device (100) according to claims 9, wherein the position determination device (100) further comprises a sound sensor adapted to register one or more distinct sounds caused by a user operating the drug administration device (200) to increase, decrease, and/or release a drug dosage as an actual action of the user and wherein the position determination device (100) is adapted to trigger deriving the data representing an actual injection location in response to registering the one or more distinct sounds.

12. The position determination device (100) according to any one of claims 1 - 11, wherein the position determination device (100) further comprises
- a memory and/or storage (160) comprising data representing at least one previous injection location,
- at least one indicator element (202; 203), adapted to signal to the user
∘ that the current location of the position determination device (100), as determined by the position determination element (110), is at a location where it is acceptable or advisable to perform an injection taking into account at least the data representing at least one previous injection location, and/or
∘ that the current location of the position determination device (100), as determined by the position determination element (110), is at a location where it is not acceptable or advisable to perform an injection taking into account at least the data representing at least one previous injection location.

13. The position determination device (100) according to any one of claims 1 - 12, wherein the position determination device (100) is further adapted to register an actual amount of administered liquid drug and/or a time-stamp when deriving data representing an actual injection location.

14. The position determination device (100) according to any one of claims 1 - 13, wherein the position determination device (100) further comprises an image sensor or other sensor element adapted to detect an amount of fluid drug being administered by the fluid drug administration device (200).

15. The position determination device (100) according to any one of claims 1 - 14, wherein the position determination device (100) is further adapted to
- recommend the anchor position and/or recommend the location of the actual injection location in response to a rotation schema taking into account one or more previously registered actual injection locations.

16. The position determination device (100) according to any one of claims 1 - 15, wherein the position determination device (100) is further adapted to
- register a starting position when the user has moved the position determination device (100) to a starting position,
- if used, registering a region in connection with the starting position defining a region suitable for receiving injections,
- registering one or more anchor points, by determining their relative position in relation to the starting position, and for each anchor point registering a region defining a region associated with the given anchor point being suitable for receiving injections.

17. The position determination device (100) according to any one of claims 1 - 16, wherein the fluid drug is one selected from the group consisting of: insulin, drugs for multiple sclerosis therapies with interferons and immune suppressives, drugs for hormone therapies, drugs for growth hormone therapies, and drugs for anti-/hormone followup treatments to breast or prostate cancer.

18. The position determination device (100) according to any one of claims 1, 8 - 9, 12 - 13, and 15 - 17, wherein the position determination device (100) is integrated with a fluid drug administration device (200) and wherein the derived data representing an actual injection location is made available to at least another electric element of the fluid drug administration device (200).

19. The position determination device (100) according to any one of claims 1 - 18, wherein the position determination device (100) is for determining and registering an injection location of self-administered fluid drug administration on a user, or the position determination device (100) is for determining and registering an injection location of administered fluid drug administration on a user administered by a medical professional.

20. The position determination device (100) according to any one of claims 1 - 19, wherein the starting movement vector is a zero-length movement vector.

## Patentansprüche

1. Positionsbestimmungsvorrichtung (100) zum Bestimmen und Registrieren eines Injektionsortes einer verabreichten Fluidmedikamentverabreichung an einem Benutzer, wobei die Positionsbestimmungsvorrichtung (100) Folgendes umfasst:
- ein Positionsbestimmungselement (110), das angepasst ist, um einen aktuellen Ort der Positionsbestimmungsvorrichtung (100) zu bestimmen, wobei das Positionsbestimmungselement (110) mindestens ein Gyroskop und mindesten einen Beschleunigungsmesser umfasst, und
- eine oder mehrere Verarbeitungseinheiten (150), die angepasst sind, um einen tatsächlichen Injektionsort darstellende Daten basierend auf einem von dem Positionsbestimmungselement (110) bestimmten aktuellen Ort abzuleiten,
wobei die Positionsbestimmungsvorrichtung (100) angepasst ist, um
- eine Startposition in Reaktion darauf, dass der Benutzer die Positionsbestimmungsvorrichtung (100) in eine vorgegebene Startposition bewegt, zu registrieren,
**dadurch gekennzeichnet, dass** die Positionsbestimmungsvorrichtung (100) weiter angepasst ist, um
- einen Ort einer Ankerposition in Reaktion darauf, dass das Positionsbestimmungselement (110) die Bewegung von der Startposition in die Ankerposition bestimmt, zu registrieren,
- einen Ort des tatsächlichen Injektionsortes in Reaktion darauf, dass das Positionsbestimmungselement (110) die Bewegung von der Ankerposition zu dem Injektionsort bestimmt, zu registrieren,
- das Positionsbestimmungselement (110) neu einzustellen, um einen Startbewegungsvektor in der Startposition darzustellen,
- eine interne Darstellung des Positionsbestimmungselements (110) zur Erzeugung eines dreidimensionalen Bewegungsvektors in Reaktion auf die von der Startposition bewegten Distanz zu aktualisieren,
- wenn die Positionsbestimmungsvorrichtung (100) in die Ankerposition bewegt wird, den dreidimensionalen Bewegungsvektor mit kalibrierten Vektoren zu vergleichen, um zu bestimmen, in welcher Ankerposition sich die Positionsbestimmungsvorrichtung (100) befindet, und
- eine genaue Position einer Injektionsstelle in Reaktion darauf, dass der Benutzer das Positionsbestimmungselement (110) von der Ankerposition zu einer endgültigen Injektionsstelle bewegt, zu bestimmen.

2. Positionsbestimmungsvorrichtung (100) nach Anspruch 1, wobei die eine oder die mehreren Verarbeitungseinheiten (150) angepasst sind, um die einen tatsächlichen Injektionsort darstellenden Daten basierend auf einem von dem Positionsbestimmungselement (110) bestimmten aktuellen Ort in Reaktion darauf, dass der Benutzer das Fluidmedikament unter Verwendung einer Fluidmedikamentverabreichungsvorrichtung (200) verabreicht, abzuleiten.

3. Positionsbestimmungsvorrichtung (100) nach einem der Ansprüche 1-2, wobei
- die Positionsbestimmungsvorrichtung (100) weiter angepasst ist, um an der Fluidmedikamentverabreichungsvorrichtung (200) gesichert zu werden.

4. Positionsbestimmungsvorrichtung (100) nach Anspruch 3, wobei die Positionsbestimmungsvorrichtung (100) eine allgemein hohle zylindrische Form mit einer Öffnung oder einem Ausschnitt (240) aufweist, angepasst, um mindestens einen Teil der Fluidmedikamentverabreichungsvorrichtung (200), wenn daran gesichert, aufzunehmen.

5. Positionsbestimmungsvorrichtung (100) nach einem der Ansprüche 3-4, wobei die Positionsbestimmungsvorrichtung (100) einen Einrastmechanismus (701) umfasst, der angepasst ist, um lösbar in einen dazu passenden oder entsprechenden Einrastmechanismus (801) der Flüssigmedikamentverabreichungsvorrichtung (200) einzugreifen und dadurch die Positionsbestimmungsvorrichtung (100) an einem vorgegebenen Ort auf der Medikamentverabreichungsvorrichtung und/oder in einer vorgegebenen Ausrichtung in Bezug auf diese zu befestigen.

6. Positionsbestimmungsvorrichtung (100) nach Anspruch 5, wobei der Einrastmechanismus (701) einem Einrastmechanismus einer Kappe (210) der Medikamentverabreichungsvorrichtung (200) entspricht.

7. Positionsbestimmungsvorrichtung (100) nach einem der Ansprüche 5 oder 6, wobei der Einrastmechanismus (701) weiter angepasst ist, um lösbar in einen dazu passenden oder entsprechenden Einrastmechanismus (801) einer Kappe (210) der Flüssigmedikamentverabreichungsvorrichtung (200) einzugreifen und dadurch der Kappe (210) zu ermöglichen, an der Positionsbestimmungsvorrichtung (100) befestigt zu werden.

8. Positionsbestimmungsvorrichtung (100) nach einem der Ansprüche 1-7, wobei
- die Positionsbestimmungsvorrichtung (100) weiter eine drahtlose Kommunikationseinheit (120) umfasst, die angepasst ist, um die einen tatsächlichen Injektionsort darstellenden abgeleiteten Daten automatisch an eine separate elektrische Vorrichtung (200; 300) zu übermitteln.

9. Positionsbestimmungsvorrichtung (100) nach einem der Ansprüche 1-8, wobei
- eine tatsächliche Aktion des Benutzers, die Fluidmedikamentverabreichungsvorrichtung (200) zu aktivieren, um das Fluidmedikament zu verabreichen, das Ableiten der einen tatsächlichen Injektionsort darstellenden Daten auslöst.

10. Positionsbestimmungsvorrichtung (100) nach Anspruch 9, wobei ein mechanischer oder Fernbedienungssensor an einer Aktivierungstaste der Fluidmedikamentverabreichungsvorrichtung (200) direkt befestigt oder in Verbindung damit befestigt ist, wobei der mechanische oder Fernbedienungssensor angepasst ist, um Bewegung der Aktivierungstaste der Fluidmedikamentverabreichungsvorrichtung (200) zu detektieren.

11. Positionsbestimmungsvorrichtung (100) nach Anspruch 9, wobei die Positionsbestimmungsvorrichtung (100) weiter einen Tonsensor umfasst, der angepasst ist, um einen oder mehrere einzelne Töne, die von einem Benutzer verursacht werden, der die Medikamentverabreichungsvorrichtung (200) bedient, um eine Medikamentendosierung zu erhöhen, zu verringern und/oder freizusetzen, als tatsächliche Aktion des Benutzers zu registrieren, und wobei die Positionsbestimmungsvorrichtung (100) angepasst ist, um das Ableiten der einen tatsächlichen Injektionsort darstellenden Daten in Reaktion auf das Registrieren des einen oder der mehreren einzelnen Töne auszulösen.

12. Positionsbestimmungsvorrichtung (100) nach einem der Ansprüche 1-11, wobei die Positionsbestimmungsvorrichtung (100) weiter Folgendes umfasst:
- einen Speicherort und/oder Speicher (160), der mindestens einen vorherigen Injektionsort darstellende Daten umfasst;
- mindestens ein Anzeigeelement (202; 203), das angepasst ist, um dem Benutzer zu signalisieren,
∘ dass sich der aktuelle Ort der Positionsbestimmungsvorrichtung (100), wie von dem Positionsbestimmungselement (110) bestimmt, an einem Ort befindet, an dem es akzeptabel oder ratsam ist, unter Berücksichtigung mindestens der Daten, die mindestens einen vorherigen Injektionsort darstellen, eine Injektion durchzuführen, und/oder
∘ dass sich der aktuelle Ort der Positionsbestimmungsvorrichtung (100), wie von dem Positionsbestimmungselement (110) bestimmt, an einem Ort befindet, an dem es nicht akzeptabel oder ratsam ist, unter Berücksichtigung mindestens der Daten, die mindestens einen vorherigen Injektionsort darstellen, eine Injektion durchzuführen.

13. Positionsbestimmungsvorrichtung (100) nach einem der Ansprüche 1-12, wobei die Positionsbestimmungsvorrichtung (100) weiter angepasst ist, um eine tatsächliche Menge an verabreichtem Flüssigmedikament und/oder eine Zeitmarkierung für den Zeitpunkt des Ableitens von einen tatsächlichen Injektionsort darstellenden Daten zu registrieren.

14. Positionsbestimmungsvorrichtung (100) nach einem der Ansprüche 1-13, wobei die Positionsbestimmungsvorrichtung (100) weiter einen Bildsensor oder ein anderes Sensorelement, das angepasst ist, um eine Menge an von der Fluidmedikamentverabreichungsvorrichtung (200) verabreichtem Fluidmedikament zu detektieren, umfasst.

15. Positionsbestimmungsvorrichtung (100) nach einem der Ansprüche 1-14, wobei die Positionsbestimmungsvorrichtung (100) weiter angepasst ist, um
- die Ankerposition zu empfehlen und/oder den Ort des tatsächlichen Injektionsortes in Reaktion auf ein Rotationsschema unter Berücksichtigung einer oder mehrerer zuvor registrierter tatsächlicher Injektionsorte zu empfehlen.

16. Positionsbestimmungsvorrichtung (100) nach einem der Ansprüche 1-15, wobei die Positionsbestimmungsvorrichtung (100) weiter angepasst ist, um
- eine Startposition zu registrieren, wenn der Benutzer die Positionsbestimmungsvorrichtung (100) in eine Startposition bewegt hat,
- wenn verwendet, einen Bereich in Verbindung mit der Startposition zu registrieren, der eine für das Aufnehmen von Injektionen geeigneten Bereich definiert,
- einen oder mehrere Ankerpunkte durch Bestimmen ihrer relativen Position in Bezug auf die Startposition zu registrieren und für jeden Ankerpunkt einen Bereich zu registrieren, der einen mit dem gegebenen Ankerpunkt verknüpften Bereich definiert, der für das Aufnehmen von Injektionen geeignet ist.

17. Positionsbestimmungsvorrichtung (100) nach einem der Ansprüche 1-16, wobei das Fluidmedikament eines ist, das ausgewählt ist aus der Gruppe, bestehend aus: Insulin, Medikamenten für Multiple-Sklerose-Therapien mit Interferonen und Immunsuppressiva, Medikamenten für Hormontherapien, Medikamenten für Wachstumshormontherapien und Medikamenten für (Anti)-Hormon-Nachbehandlungen bei Brust- oder Prostatakrebs.

18. Positionsbestimmungsvorrichtung (100) nach einem der Ansprüche 1, 8-9, 12-13 und 15-17, wobei die Positionsbestimmungsvorrichtung (100) in eine Fluidmedikamentverabreichungsvorrichtung (200) integriert ist und wobei die einen tatsächlichen Injektionsort darstellenden abgeleiteten Daten mindestens einem anderen elektrischen Element der Fluidmedikamentverabreichungsvorrichtung (200) zur Verfügung gestellt werden.

19. Positionsbestimmungsvorrichtung (100) nach einem der Ansprüche 1-18, wobei die Positionsbestimmungsvorrichtung (100) dem Bestimmen und Registrieren eines Injektionsortes der selbstverabreichten Fluidmedikamentverabreichung an einem Benutzer dient oder die Positionsbestimmungsvorrichtung (100) dem Bestimmen und Registrieren eines Injektionsortes der verabreichten Fluidmedikamentverabreichung an einem Benutzer, verabreicht durch eine medizinische Fachkraft, dient.

20. Positionsbestimmungsvorrichtung (100) nach einem der Ansprüche 1-19, wobei der Startbewegungsvektor ein Nulllängenbewegungsvektor ist.

## Revendications

1. Dispositif de détermination de position (100) pour déterminer et enregistrer un emplacement d'injection d'administration de médicament sous forme liquide administré à un utilisateur, dans lequel le dispositif de détermination de position (100) comprend
- un élément de détermination de position (110) conçu pour déterminer un emplacement actuel du dispositif de détermination de position (100), l'élément de détermination de position (110) comprenant au moins un gyroscope et au moins un accéléromètre, et
- une ou plusieurs unités de traitement (150) conçues pour dériver des données représentant un emplacement d'injection actuel sur la base d'un emplacement actuel déterminé par l'élément de détermination de position (110),
dans lequel le dispositif de détermination de position (100) est conçu pour
- enregistrer une position de départ en réponse au déplacement par l'utilisateur du dispositif de détermination de position (100) vers une position de départ prédéterminée,
**caractérisé en ce que** le dispositif de détermination de position (100) est en outre conçu pour
- enregistrer un emplacement d'une position d'ancrage en réponse à la détermination par l'élément de détermination de position (110) du mouvement entre la position de départ et la position d'ancrage,
- enregistrer un emplacement de l'emplacement d'injection actuel en réponse à la détermination par l'élément de détermination de position (110) du mouvement entre la position d'ancrage et l'emplacement d'injection,
- réinitialiser l'élément de détermination de position (110) pour représenter un vecteur de mouvement de départ au niveau de la position de départ,
- mettre à jour une représentation interne de l'élément de détermination de position (110) pour produire un vecteur de mouvement en trois dimensions en réponse à la distance parcourue depuis la position de départ,
- lorsque le dispositif de détermination de position (100) est déplacé vers la position d'ancrage, comparer ensuite le vecteur de mouvement en trois dimensions à des vecteurs étalonnés pour déterminer au niveau de quelle position d'ancrage se trouve le dispositif de détermination de position (100), et
- déterminer une position exacte d'un site d'injection en réponse au déplacement par l'utilisateur de l'élément de détermination de position (110) entre la position d'ancrage et un site d'injection final.

2. Dispositif de détermination de position (100) selon la revendication 1, dans lequel les une ou plusieurs unités de traitement (150) sont conçues pour dériver les données représentant un emplacement d'injection actuel sur la base d'un emplacement actuel déterminé par l'élément de détermination de position (110) en réponse à l'administration par l'utilisateur du médicament sous forme liquide en utilisant un dispositif d'administration de médicament sous forme liquide (200).

3. Dispositif de détermination de position (100) selon l'une quelconque des revendications 1 et 2, dans lequel
- le dispositif de détermination de position (100) est en outre conçu pour être fixé au dispositif d'administration de médicament sous forme liquide (200).

4. Dispositif de détermination de position (100) selon la revendication 3, dans lequel le dispositif de détermination de position (100) a une forme cylindrique généralement creuse avec une ouverture ou une découpe (240) conçue pour recevoir au moins une partie du dispositif d'administration de médicament sous forme liquide (200) lorsqu'elle est fixée à celui-ci.

5. Dispositif de détermination de position (100) selon l'une quelconque des revendications 3 et 4, dans lequel le dispositif de détermination de position (100) comprend un mécanisme de verrouillage (701) conçu pour venir en prise de manière libérable avec un mécanisme d'accouplement ou de verrouillage correspondant (801) du dispositif d'administration de médicament sous forme liquide (200) fixant ainsi le dispositif de détermination de position (100) au niveau d'un emplacement prédéterminé sur le dispositif d'administration de médicament et/ou selon une orientation prédéterminée par rapport au dispositif d'administration de médicament.

6. Dispositif de détermination de position (100) selon la revendication 5, dans lequel le mécanisme de verrouillage (701) correspond à un mécanisme de verrouillage d'un capuchon (210) du dispositif d'administration de médicament (200).

7. Dispositif de détermination de position (100) selon la revendication 5 ou 6, dans lequel le mécanisme de verrouillage (701) est en outre conçu pour venir en prise de manière libérable avec un mécanisme d'accouplement ou de verrouillage correspondant (801) d'un capuchon (210) du dispositif d'administration de médicament sous forme liquide (200) permettant ainsi au capuchon (210) d'être fixé au dispositif de détermination de position (100).

8. Dispositif de détermination de position (100) selon l'une quelconque des revendications 1 à 7, dans lequel
- le dispositif de détermination de position (100) comprend en outre une unité de communication sans fil (120) conçue pour transmettre automatiquement à un dispositif électrique séparé (200; 300) les données dérivées représentant un emplacement d'injection actuel.

9. Dispositif de détermination de position (100) selon l'une quelconque des revendications 1 à 8, dans lequel
- une action actuelle de l'utilisateur activant le dispositif d'administration de médicament sous forme liquide (200) pour administrer le médicament sous forme liquide déclenche la dérivation des données représentant un emplacement d'injection actuel.

10. Dispositif de détermination de position (100) selon la revendication 9, dans lequel un capteur mécanique ou distant est fixé directement à un bouton d'activation du dispositif d'administration de médicament sous forme liquide (200) ou est fixé en connexion avec celui-ci, dans lequel le capteur mécanique ou distant est conçu pour détecter un mouvement du bouton d'activation du dispositif d'administration de médicament sous forme liquide (200).

11. Dispositif de détermination de position (100) selon la revendication 9, dans lequel le dispositif de détermination de position (100) comprend en outre un capteur sonore conçu pour enregistrer un ou plusieurs sons distincts résultant de l'actionnement du dispositif d'administration de médicament (200) par un utilisateur pour augmenter, diminuer et/ou libérer une dose de médicament comme action actuelle de l'utilisateur et dans lequel le dispositif de détermination de position (100) est conçu pour déclencher la dérivation des données représentant un emplacement d'injection actuel en réponse à l'enregistrement des un ou plusieurs sons distincts.

12. Dispositif de détermination de position (100) selon l'une quelconque des revendications 1 à 11, dans lequel le dispositif de détermination de position (100) comprend en outre
- une mémoire et/ou un stockage (160) comprenant des données représentant au moins un emplacement d'injection précédent,
- au moins un élément indicateur (202 ; 203), conçu pour signaler à l'utilisateur
- que l'emplacement actuel du dispositif de détermination de position (100), tel que déterminé par l'élément de détermination de position (110), se trouve au niveau d'un emplacement où il est acceptable ou recommandé d'effectuer une injection en tenant compte au moins des données représentant au moins un emplacement d'injection précédent, et/ou
- que l'emplacement actuel du dispositif de détermination de position (100), tel que déterminé par l'élément de détermination de position (110), se trouve au niveau d'un emplacement où il n'est pas acceptable ni recommandé d'effectuer une injection en tenant compte au moins des données représentant au moins un emplacement d'injection précédent.

13. Dispositif de détermination de position (100) selon l'une quelconque des revendications 1 à 12, dans lequel le dispositif de détermination de position (100) est en outre conçu pour enregistrer une quantité actuelle de médicament sous forme liquide administré et/ou un horodatage lors de la dérivation de données représentant un emplacement d'injection actuel.

14. Dispositif de détermination de position (100) selon l'une quelconque des revendications 1 à 13, dans lequel le dispositif de détermination de position (100) comprend en outre un capteur d'image ou autre élément capteur conçu pour détecter une quantité de médicament sous forme liquide administrée par le dispositif d'administration de médicament sous forme liquide (200).

15. Dispositif de détermination de position (100) selon l'une quelconque des revendications 1 à 14, dans lequel le dispositif de détermination de position (100) est en outre conçu pour
- recommander la position d'ancrage et/ou recommander l'emplacement de l'emplacement d'injection actuel en réponse à un schéma de rotation tenant compte d'un ou plusieurs emplacements d'injection actuels précédemment enregistrés.

16. Dispositif de détermination de position (100) selon l'une quelconque des revendications 1 à 15, dans lequel le dispositif de détermination de position (100) est en outre conçu pour
- enregistrer une position de départ lorsque l'utilisateur a déplacé le dispositif de détermination de position (100) vers une position de départ,
- s'il est utilisé, enregistrer une région en connexion avec la position de départ définissant une région appropriée pour recevoir des injections,
- enregistrer un ou plusieurs points d'ancrage en déterminant leur position relative par rapport à la position de départ, et pour chaque point d'ancrage enregistrer une région définissant une région associée au point d'ancrage donné approprié pour recevoir des injections.

17. Dispositif de détermination de position (100) selon l'une quelconque des revendications 1 à 16, dans lequel le médicament sous forme liquide est un choisi dans le groupe constitué de : insuline, médicaments pour traitements de la sclérose en plaques comprenant des interférons et des immunosuppresseurs, médicaments pour traitements hormonaux, médicaments pour traitement par l'hormone de croissance et médicaments pour traitements hornomaux/anti-hormonaux de suivi du cancer du sein ou de la prostate.

18. Dispositif de détermination de position (100) selon l'une quelconque des revendications 1, 8 et 9, 12 et 13 et 15 à 17, dans lequel le dispositif de détermination de position (100) est intégré à un dispositif d'administration de médicament sous forme liquide (200) et dans lequel les données dérivées représentant un emplacement d'injection actuel sont rendues disponibles à au moins un autre élément électrique du dispositif d'administration de médicament sous forme liquide (200).

19. Dispositif de détermination de position (100) selon l'une quelconque des revendications 1 à 18, dans lequel le dispositif de détermination de position (100) est destiné à déterminer et à enregistrer un emplacement d'injection d'administration de médicament sous forme liquide auto-administré sur un utilisateur, ou le dispositif de détermination de position (100) est destiné à déterminer et enregistrer un emplacement d'injection d'administration de médicament sous forme liquide administré sur un utilisateur, administré par un professionnel médical.

20. Dispositif de détermination de position (100) selon l'une quelconque des revendications 1 à 19, dans lequel le vecteur de mouvement de départ est un vecteur de mouvement de longueur zéro.
